(19) Europäisches Patentamt
European Patent Office
Office européen des brevets

(11) Publication number: **0 066 406 A1**

(12) **EUROPEAN PATENT APPLICATION**

(21) Application number: 82302511.9

(22) Date of filing: 18.05.82

(51) Int. Cl.³: C 07 D 499/70
A 61 K 31/43
//C07D277/46

(30) Priority: 28.05.81 GB 8116390

(43) Date of publication of application: 08.12.82 Bulletin 82/49

(84) Designated Contracting States: CH DE FR GB IT LI NL

(71) Applicant: BEECHAM GROUP PLC
Beecham House Great West Road
Brentford Middlesex(GB)

(72) Inventor: Stachulski, Andrew Valentine
Cornerways Cliftonville
Dorking Surrey(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

(54) **Beta-lactam antibiotics.**

(57) A compound of formula (I) or a pharmaceutically acceptable salt or *in vivo* hydrolysable ester thereof:

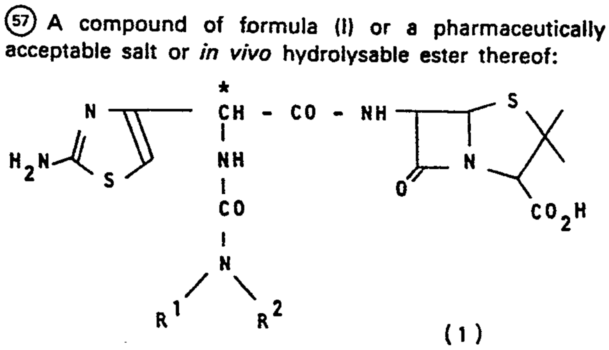

(1)

wherein $R^1$ is hydrogen and $R^2$ is an optionally substituted five- or six-membered heterocyclic group containing one or two nitrogen heteroatoms; or $R^1$ and $R^2$ together with the nitrogen atom to which they are attached form an optionally substituted five- or six-membered heterocyclic group containing one or two nitrogen heteroatoms.

EP 0 066 406 A1

Croydon Printing Company Ltd.

## β-Lactam Antibiotics

This invention relates to a class of penicillins which have antibacterial activity and are of value in the treatment of infections in animals, especially mammals, including man, caused by a wide range of organisms, particularly Gram-negative organisms. In particular, the invention relates to a class of penicillins having an aminothiazole group in the side chain. The invention also relates to a process for the preparation of such compounds and to pharmaceutical compositions comprising them.

According to the present invention there is provided a compound of formula (I) or a pharmaceutically acceptable salt or *in vivo* hydrolysable ester thereof:

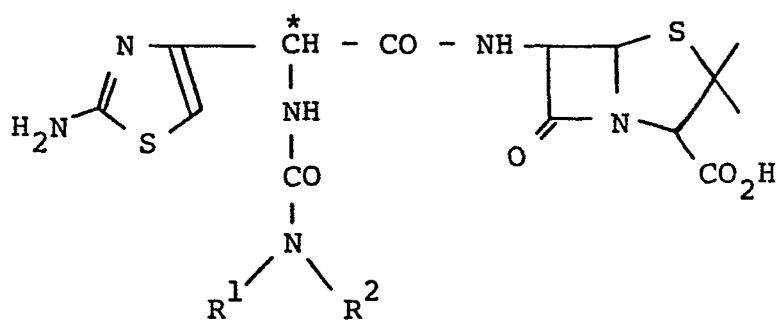

(I)

wherein $R^1$ is hydrogen and $R^2$ is an optionally substituted five- or six-membered heterocyclic group containing one or two nitrogen heteroatoms; or $R^1$ and $R^2$ together with the nitrogen atom to which they are attached form an optionally substituted five- or six-membered heterocyclic group containing one or two nitrogen heteroatoms.

The compounds of the present invention include the pharmaceutically acceptable esters of compound (I) which hydrolyse readily in the human body to produce the parent acid, for example, acyloxyalkyl groups, such as acetoxymethyl, pivaloyloxymethyl, α-acetoxyethyl, α-acetoxymethyl and α-pivaloyloxyethyl groups; alkoxycarbonyloxyalkyl groups, such as ethoxycarbonyloxymethyl and α-ethoxycarbonyloxyethyl; dialkylaminoalkyl groups such as dimethylaminomethyl, dimethylaminoethyl, diethylaminomethyl or diethylaminoethyl; and lactone groups such as phthalidyl or dimethoxyphthalidyl.

The compounds of the present invention contain both an amino group and a carboxyl group and may, therefore, exist as the zwitterion or may form salts with suitable acids or bases.

Suitable salts of the carboxy group of the compound of formula (I) include metal salts e.g. aluminium, alkali metal salts such as sodium or potassium alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as bicyclohexylamine, or with procaine, dibenzylamine, N,N-dibenzylethylenediamine, 1-ephenamine, N-ethylpiperidine,

N-benzyl-β-phenethylamine, dehydroabietylamine, N,N'-bisdehydroabietylamine, ethylenediamine, or bases of the pyridine type such as pyridine, collidine or quinoline, or other amines which have been used to form salts with known penicillins.

The carbon atom marked * in formula (I) is asymmetric so that the compounds may exist as two optically active diastereoisomers. In general that prepared from the D-side chain exhibits the highest antibacterial activity.

Suitable substituents for the five- or six-membered heterocyclic group include the alkyl, alkenyl, alkynyl, cycloalkyl or cycloalkenyl group, optionally substituted phenyl, oxo, the hydroxy group optionally substituted by alkyl, alkenyl, cycloalkyl, phenyl, pyridyl, pyrimidyl or benzyl, the optionally substituted mercapto group, the alkylsulphenyl group, or the amino group optionally substituted by an alkyl, alkenyl, cycloalkyl, phenyl, substituted phenyl or benzyl group. Alternatively two substituents on the ring may form the residue of a further carbocyclic or heterocyclic ring.

One preferred subgroup within the present invention provides a compound of formula (II) or a pharmaceutically acceptable salt or in vivo hydrolysable ester thereof:

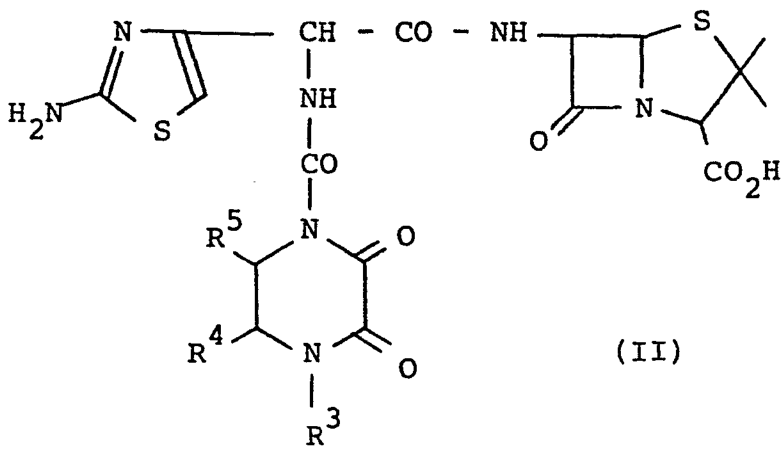

(II)

wherein $R^3$ represents hydrogen or $C_{1-6}$ alkyl; $R^4$ and $R^5$ are the same or different and represent hydrogen, $C_{1-6}$ alkyl, halogen, amino, hydroxy or $C_{1-6}$ alkoxy.

Suitable $C_{1-6}$ alkyl groups for the groups $R^3$, $R^4$ and $R^5$ include methyl, ethyl, n- and iso-propyl, n, sec-, iso- and tert-butyl. Preferably $R^3$ is ethyl. Preferably $R^4$ and $R^5$ are hydrogen.

Specific compounds within this invention include the following:

6-[DL-α-(4-ethyl-2,3-dioxopiperazine-1-carbonylamino)2-aminothiazol-4-acetamido]penicillanic acid or a pharmaceutically acceptable salt or in vivo hydrolysable ester thereof;
6-[D-α-(4-ethyl-2,3-dioxopiperazine-1-carbonylamino)2-aminothiazol-4-acetamido]penicillanic acid or a pahramceutically acceptable salt or in vivo hydrolysable ester thereof.

The compounds of formula (I) may be prepared by reacting a compound of formula (III):

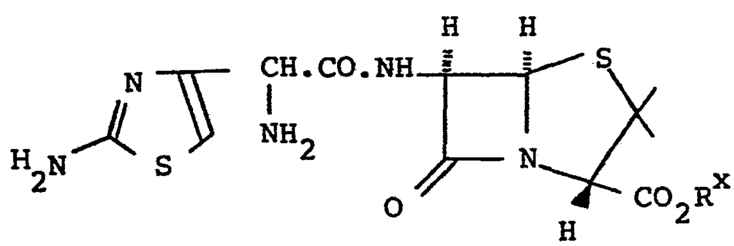

(III)

wherein the α-amino group is optionally substituted with a group which permits acylation to take place, and any reactive groups on the aminothiazole group may be protected, and $R^x$ is hydrogen or a carboxyl-blocking group, with an N-acylating derivative of an acid of formula (IV):

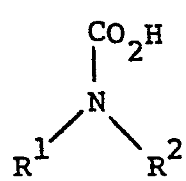

(IV)

wherein $R^1$ and $R^2$ are as defined with respect to formula (I) above and any reactive groups may be protected; and thereafter, if necessary, carrying out one or more of the following steps:

(i) removing any carboxyl-blocking group $R^x$;

(ii) removing any protecting groups on the side chain group;

(iii) converting the product into a salt or <u>in vivo</u> hydrolysable ester thereof.

Suitable groups which permit acylation to take place and which are optionally present on the amino group of the starting material of the formula (II) include N-silyl, N-stannyl and N-phosphorus groups, for example, trialkylsilyl groups such as trimethylsilyl, trialkyltin groups such as tri-*n*-butyltin, groups of formula $-P.R^aR^b$ wherein $R^a$ is an alkyl, haloalkyl, aryl, aralkyl, alkoxy, haloalkoxy, aryloxy, aralkyloxy or dialkylamino group, $R^b$ is the same as $R^a$ or is halogen or $R^a$ and $R^b$ together form a ring; suitable such phosphorus groups being:

$-P(OC_2H_5)_2$ , $-P(C_2H_5)_2$ , 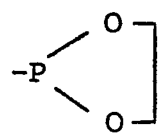 and 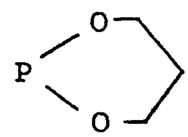 .

Suitable carboxyl-blocking derivatives for the group $-CO_2R^x$ in formula (II) include salts and ester derivatives of the carboxylic acid. The derivative is preferably one which may readily be cleaved at a later stage of the reaction. Suitable salts include metal salts, such as those with sodium, potassium and lithium, and tertiary amine salts, such as those with tri-lower-alkylamines, N-ethylpiperidine, 2,6-lutidine, pyridine, N-methylpyrrolidine, dimethylpiperazine. A preferred salt is with triethylamine.

Suitable ester-forming carboxyl-blocking groups are those which may be removed under conventional conditions. Such groups for $R^x$ include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, such as described above, an oxime radical of formula $-N=CHR^o$ where $R^o$ is aryl or heterocyclic, or an in vivo hydrolysable ester radical such as defined above.

The carboxyl group may be regenerated from any of the above esters by usual methods appropriate to the particular $R^x$ group, for example, acid - and base - catalysed hydrolysis, or by enzymically - catalysed hydrolysis, or by hydrogenation.

A reactive N-acylating derivative of the acid (III) is employed in the above process. The choice of reactive derivative will, of course, be influenced by the chemical nature of the substituents of the acid.

Suitable N-acylating derivatives include an acid halide, preferably the acid chloride or bromide. Acylation with an acid halide may be affected in the presence of an acid binding agent, for example tertiary amine (such as triethylamine or dimethylaniline), an inorganic base (such as calcium carbonate or sodium carbonate) or an oxirane, which binds hydrogen halide liberated in the acylation reaction. The oxirane is preferably a $(C_{1-6})$-1,2,alkylene oxide - such as ethylene oxide or propylene oxide. The

acylation reaction using an acid halide may be carried out at a temperature in the range $-50^{o}C$ to $+50^{o}C$, preferably $-20^{o}$ to $+20^{o}C$, in aqueous or non-aqueous media such as aqueous acetone, aqueous tetrahydroform, ethyl acetate, dimethylacetamide, dimethylformamide, acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate.

The compounds of formula (I) may also be prepared by reacting a compound of formula (V):

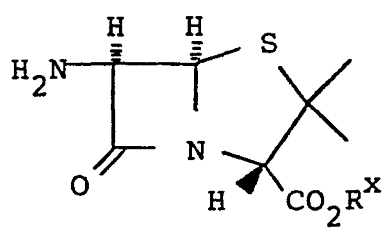

(V)

wherein the amino group is optionally substituted with a group which permits acylation to take place and $R^x$ is as defined with respect to formula (II) above, with an N-acylating derivative of an acid of formula (VI):

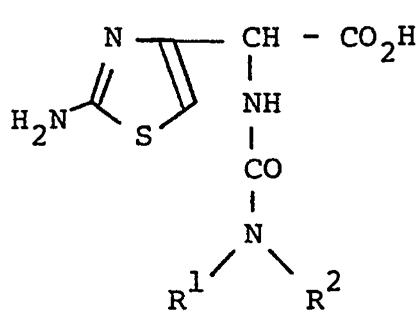

(VI)

wherein $R^1$ and $R^2$ are as defined with respect to formula (I) and any reactive groups therein may be protected; and thereafter, if necessary, carrying out one or more of the following steps:

(i) removing any carboxyl-blocking group $R^x$;
(ii) removing any protecting groups on the side chain group;
(iii) converting the product into a salt or in vivo hydrolysable ester thereof.

The antibiotic compounds according to the invention may be formulated for administration in any convenient way for use in human or veterinary medicine, by analogy with other antibiotics, and the invention therefore includes within its scope a pharmaceutical composition comprising a compound of formula (I) above together with a pharmaceutical carrier or excipient.

The compositions may be formulated for administration by any route, such as oral, topical or parenteral. The compositions may be in the form of tablets, capsules, powders, granules, lozenges, creams or liquid preparations, such as oral or sterile parenteral solutions or suspensions.

Tablets and capsules for oral administration may be in unit dose presentation form, and may contain conventional excipients such as binding agents, for example syrup, acacia, gelatin, sorbitol, tragacanth or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate.

The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, methyl cellulose, glucose syrup, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilising the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in

the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance, 1500 mg per day depending on the route and frequency of administration.

The compound of formula (I) may be the sole therapeutic agent in the compositions of the invention or a combination with other antibiotics or with a β-lactamase inhibititor may be employed. Advantageously, the compositions also comprise a compound of formula (VII) or a pharmaceutically acceptable salt or ester thereof:

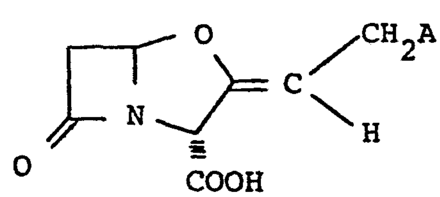

(VII)

wherein A is hydroxyl, substituted hydroxyl, thiol, substituted thiol, amino, mono- or di-hydrocarbyl-substituted amino, or mono- or di-acylamino.

A further advantageous composition comprises a compound of formula (I) or a pharmaceutically acceptable salt or *in vivo* hydrolysable ester thereof together with a compound of formula (VIII) or a pharmaceutically acceptable salt or *in vivo* hydrolysable ester thereof:

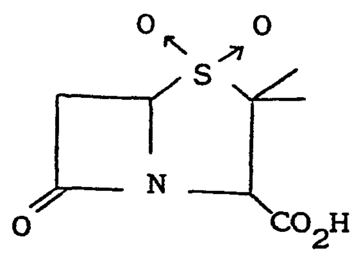

(VIII)

The present invention also provides a method of treating bacterial infections in animals, in particular humans or domestic mammals, which comprises the administration of composition of this invention.

The following Examples illustrate the preparation of the compounds of this invention.

Example 1

6-[DL-α-(4-Ethyl-2,3-dioxopiperazin-1-carbonylamino)-2-aminothiazol-4-acetamido]penicillanic acid

(a) Ethyl anti-α-methoxyimino-N,N'-bis-p-nitrobenzyloxycarbonyl-2-imino-4-thiazoline-4-acetate

Ethyl anti-α-methoxyimino 2-aminothiazol-4-acetate (ref. below) (1.6 g, 7 mmole) was dissolved in purified methylene chloride (15 ml) and stirred vigorously at room temperature with 1.0 M sodium hydrogen carbonate solution (25 ml). A solution of 4-nitrobenzylchloro-formate (3.66 g, 17 mmole) in methylene chloride (4 ml) was added in one portion and the two-phase mixture was stirred vigorously at room temperature for 2h. The product precipitated as a pale yellow solid, assisted by the addition of a little ether (1.88 g, 47%); mp 110-115$^{o}$; Rf 0.70 in methanol: chloroform, 1:9.

δ [$(CD_3)_2SO$] 1.2 (3H,t,$\underline{CH_3}CH_2O$), 4.0 (3H,s,$\underline{CH_3}O$-N), 4.25 (2H,qt,$CH_3\underline{CH_2}O$), 5.35 and 5.60 (4H,2s,2 x Ar$\underline{CH_2}$O), 7.40 (1H,s,thiazole C-5 H), 7.5-8.5 (8H,2 x AB qt, partially overlapping, aryl Hs).

Found: C, 48.95; H,3.69; N, 11.75. $C_{24}H_{21}N_5O_{11}S$ requires: C, 49.1 ; H,3.6 ; N, 11.9%.

(b) Anti-α-methoximino-2-(p-nitrobenzyloxycarbonylamino) thiazol-4-acetic acid

The preceding bis-protected ester (6.48 g, 11 mmole) was suspended in ethanol (66 ml) and stirred with a solution of potassium hydroxide (2.46 g) in water (44 ml) at ambient temperature. A clear yellow solution resulted after 1h. After 4h the solution was washed with ethyl acetate (2 x 50 ml), backwashing each with a little water, then the total aqueous phase was acidified to pH 2.0 with 5.0 M hydrochloric acid. The crude acid precipitated as a white solid and was filtered, washed with water and ether and dried. Recrystallisation from tetrahydrofuran-petrol gave, in two crops, the title acid (3.37 g, 88%); mp ill defined, >200$^o$; Rf 0.30 in chloroform:methanol: acetic acid, 17:2:1.

δ[$(CD_3)_2SO$] 4.0 (3H,s,$\underline{CH_3}$O-N), 5.40 (2H,s,Ar$\underline{CH_2}$O), 8.0 (1H,s,thiazole C-5H), 7.70 and 8.30 (4H,dd,aryl H), 12.3 (1H,brs,$D_2O$ exchanged, $CO_2H$).

Found: C, 44,20; H, 3.18; N, 14.44. $C_{14}H_{12}N_4O_7S$ requires: C, 44.20; H, 3.20; N, 14.70%.

Ref: R. Bucourt, R. Heymer, A. Lutz, L. Renasse and J. Perronet, Tetrahedron, 1978, 34, 2233.

(c) <u>6-[Anti-α-methoximino-2-(p-nitrobenzyloxycarbonylamino)thiazol-4-acetamido]penicillanic acid sodium salt</u>

The preceding protected acid (1.14 g, 3 mmole) was stirred in dry methylene chloride (15 ml) with phosphorus pentachloride (0.68 g, 3.3 mmole) for 1h at ambient temperature. The suspension was filtered, the solid washed with a little methylene chloride and dry ether, then dried in vacuo to give the acid chloride as a white solid (1.21 g, 100%), $\nu_{max}$ [Nujol] 1765 $cm^{-1}$. This was added in small portions at $0^{o}$ to a suspension of 6-amino-penicillanic acid (0.65 g, 3 mmole) in methylene chloride (12 ml) which had been stirred previously for 1h with anhydrous triethylamine (1.06 ml). The pH of the vapour above the reaction mixture was maintained at 8-9 by addition of a few further drops of triethylamine. After 1h, during which time the mixture was allowed to regain ambient temperature, the methylene chloride was evaporated then the residue was dissolved in water (30 ml) and washed with ether (2 x 20 ml). The aqueous phase was covered with ethyl acetate (20 ml), stirred vigorously and acidified to pH 2.0 with 2.0 M hydrochloric acid. The organic phase was separated, the aqueous extracted with further ethyl acetate (20 ml) and the combined organic phase washed with water and brine, then dried over anhydrous sodium sulphate. Evaporation gave the free acid form of the product, which was dissolved in ethyl acetate (30 ml). Addition of 2.0 M sodium ethyl hexanoate in methyl isobutyl ketone (1.5 ml) gave a white precipitate which was filtered, well washed with ether and dried to give the title sodium salt (1.50 g, 83%); Rf 0.70 in n-butanol: acetic acid: water, 4:1:1.

δ [$(CD_3)_2SO$] 1.50 and 1.56 (6H,2s,C-2 methyls), 3.95 (1H, s,C-3H), 4.00 (3H,s,$\underline{CH_3}$O-N), 5.36 (2H,s,Ar$\underline{CH_2}$O), 5.52 (2H,brm,ABqt on $D_2O$ exchange, C-5 and C-6 Hs), 7.69 and 8.26 (4H,dd,aryl Hs), 7.90 (1H,s,thiazole C-5H), 8.92 (1H,br d, $D_2O$ exchanged, C-6 NH);

$[\alpha]_D^{20}$ + 138.3° (c 1 in dimethyl sulphoxide); Rf 0.65 (n-butanol:ethanol:water, 7:1:2), single inhibitory zone.

(d) 6-[DL-α-Amino-2-aminothiazol-4-acetamido]penicillanic acid

The preceding sodium salt (3.60 g, 6 mmole) was converted back to its free acid form by partition between 10% aqueous citric acid and ethyl acetate. Evaporation of the solvent afforded the free acid as a semi-solid which was dissolved in ethanol (50 ml), then water (50 ml) was added. To the solution was added 10% palladium on charcoal (1.0 g), then the mixture was hydrogenated for 20 hours, adding further catalyst (1 g) after 3 hours. In the morning the catalyst was filtered and the hydrogenation resumed with fresh catalyst (1 g) for 5 hours. The catalyst was again filtered and washed well with water, then the filtrate was evaporated to dryness. Traces of catalyst were removed by redissolving in water (20 ml) and filtering. The clear yellow solution resulting was lyophilised to afford the title penicillin (1.37 g, 61%); $R_f$ 0.10 in n-butanol: acetic acid: water, 4:1:1; δ[$(CD_3)_2SO$] 1.45 and 1.55 (6H, 2s, C-2 methyls), 4.10 (1H, s, C-3 H), 4.55 and 4.60 (1H, 2s, diastereoisomeric α-CHs), 5.44 (2H, m, C-5 and C-6 Hs), 6.43 (1H, s, thiazole C-5H), 6.92 (2H, brs, thiazole C-$2NH_2$); $R_f$ 0.08 (n-butanol:ethanol:water, 7:1:2), single inhibitory zone.

(e) 6-[DL-α-(4-Ethyl-2,3-dioxopiperazin-1-carbonylamino)2-aminothiazol-4-acetamido]penicillanic acid

The preceding di-amino penicillin acid (0.74 g, 2 mmole) was dissolved in water (5 ml) and 1.0 M sodium hydrogen carbonate solution (5 ml), then tetrahydrofuran (5 ml) was added as a cosolvent. The solution was stirred at $0^o$ and a solution of 4-ethyl-2,3-dioxo-piperazine-1-carbonyl chloride (0.41 g, 2 mmole) in dry tetrahydrofuran (5 ml) was added dropwise. The pH, initially 8.0, was maintained in the range 7.0-7.5 by dropwise addition of further 1.0 M sodium hydrogen carbonate. The solution was allowed to regain ambient temperature and stirred for 1 hour, then washed with ethyl acetate (2 x 10 ml), backwashing with a little water. Acidification of the aqueous to pH 3.0 produced a small amount of yellow solid, which was filtered; this contained the desired penicillin contaminated with inorganic material. The filtrate was lyophilised and the residue stirred with dry acetone (250 ml) at ambient temperature for 1 hour. The acetone was evaporated, the residue was dissolved in water (10 ml), filtered and lyophilised to give the product (230 mg, 21%); $R_f$ 0.20 in n-butanol:acetic acid:water, 4:1:1; $\delta[(CD_3)_2SO]$ 1.16 (3H, t, $C\underline{H}_3CH_2N$), 1.43 and 1.56 (6H, 2s, C-2 methyls), 3.2-4.0 (6H, m, 3 x $C\underline{H}_2N$), 4.20 (1H, s, C-3H), 5.35 (3H, m, approx ABqt on $D_2O$ exchange, C-5 and C-6 Hs and α-CH) 6.50 (1H, s, thiazole C-5H), 6.97 (2H, brs, $D_2O$ exchanged, thiazole C-2 $N\underline{H}_2$), 8.70 and 9.60 (2H, 2d, $D_2O$ exchanged, $CHN\underline{H}$), $R_f$ 0.16 (n-butanol:ethanol:water, 7:1:2), single inhibitory zone.

## Example 2

**Synthesis of the stereoisomers of 2-(4-Ethyl-2,3-dioxo-piperazin-1-ylcarbonylamino)-2-([2-amino]thiazol-4-yl)-acetamido-6-aminopenicillanic acid**

Scheme

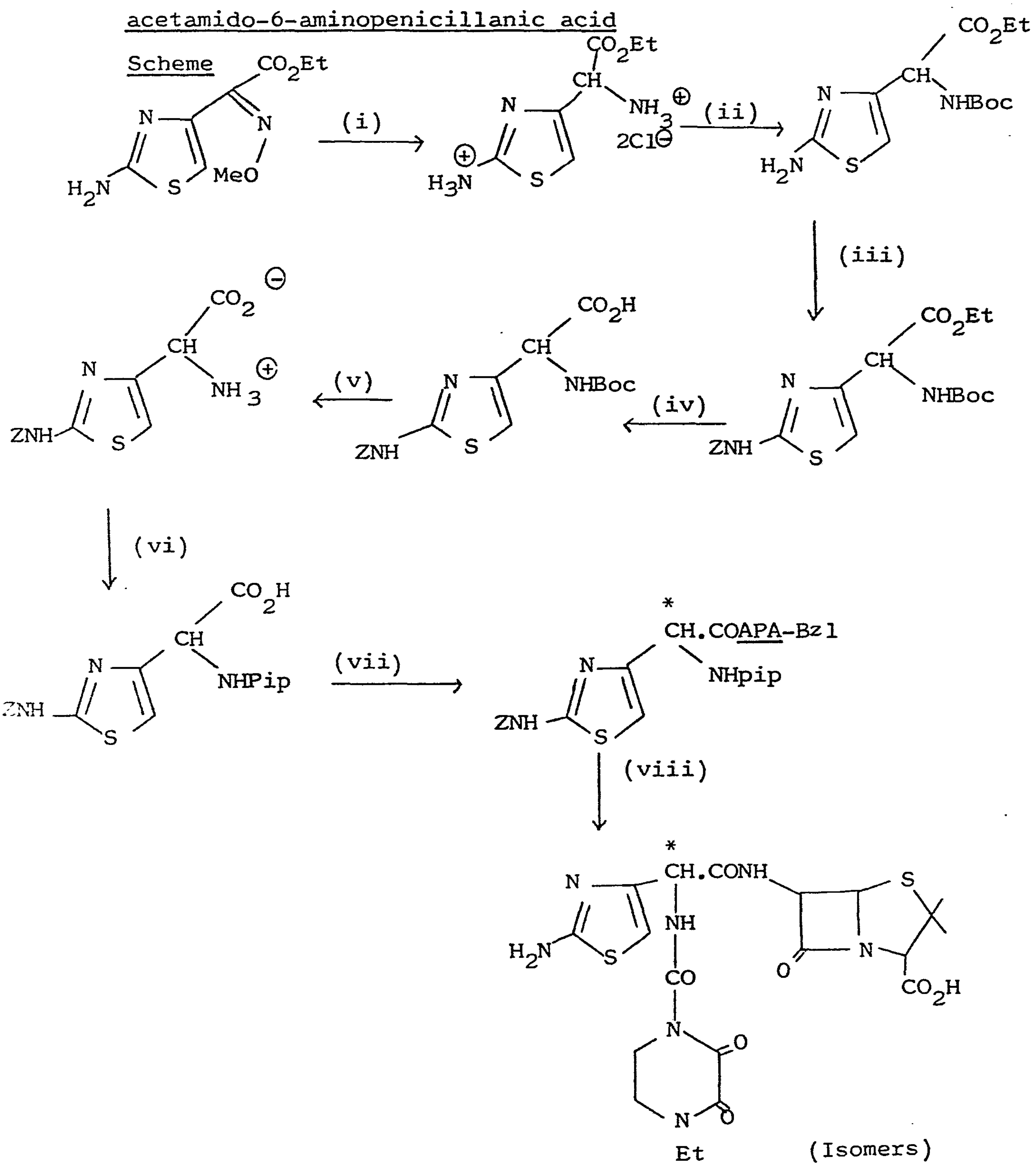

Reagents

(i) Pd-$H_2$, EtOH, aq. HCl; (ii) $(Boc)_2O$, aq. dioxan, pH9; (iii) Z-Cl, $CH_2Cl_2$/aq. $NaHCO_3$; (iv) KOH, aq. EtOH; (v) $CF_3CO_2H$; (vi) aq. THF, pH 9-10, Pip-Cl; (vii) APA-OBzl, dicyclohexylcarbodiimide, $CH_2Cl_2$; (viii) separate isomers, then $H_2$/Pd, aq. THF.

Abbreviations

Boc=t-Butoxycarbonyl; Z=benzyloxycarbonyl; THF=tetrahydrofuran; Pip=4-ethyl-2,3-dioxopiperazin-1-ylcarbonyl; APA-OBzl=benzyl 6-aminopenicillanate.

Separation of the isomers in steps (viii) was achieved by chromatography on silica gel, eluting with 2% methanol in chloroform. Satisfactory spectral and analytic data were obtained for each intermediate described. A similar sequence was performed in which the benzyloxycarbonyl protecting group was replaced by 4-nitrobenzyloxycarbonyl.

The isomeric penicillin esters isolated in step (viii) were differentiated by their n.m.r. spectra as follows:

First eluted isomer

$\delta(CDCl_3)$, 1.18 (3H, t, JHz, C$\underline{H}_3$$CH_2$N), 1.32 and 1.45 (6H, 2s, $(CH_3)_2C$), 3.49 (4H, approx. qt, J 7Hz, 2 x C$\underline{H}_2$N), 3.96 (2H, m, $CH_2$N), 4.33 (1H, s, 3-H), 5.14 and 5.25 (4H, 2s, 2 x Ph$CH_2$O), 5.47 (2H, m, 5-H and 6-H), 5.75 (1H, d, J 7Hz, ArC$\underline{H}$(NH)CO), 6.86 (1H, s, thiazole 5-H), 7.33 (10H, s, phenyls), 9.2-9.5 (1H, brs, $D_2O$ exch, thiazole 2-NH),

9.82 (1H, d, J 7Hz, $D_2O$ exch, ArCH(N<u>H</u>)CO).

<u>Second eluted isomer</u>

δ($CDCl_3$) 1.19 (3H, t, J 7Hz, C<u>H</u>$_3$CH$_2$N), 1.28 and 1.42 (6H, 2s, $(CH_3)_2C$), 3.51 (4H, approx. qt., J 7Hz, 2 x $CH_2N$), 4.02 (2H, m, $CH_2N$), 4.32 (1H, s, 3-H), 5.15 and 5.26 (4H, 2s, 2 x $PhCH_2O$), 5.50 (2H, m, 5-H and 6-H), 5.70 (1H, d, J 7Hz, ArC<u>H</u>(NH)CO), 6.80 (1H, s, thiazole 5-H), 7.35 (10H, s, phenyls), 7.65 (1H, brd, J 7Hz, $D_2O$ exch, 6-NH), 9.1-9.4 (1H, brs, $D_2O$ exch, thiazole 2-NH), 9.82 (1H, d, J 7Hz, $D_2O$ exch. ArCH(N<u>H</u>)CO).

The most diagonstic resonance is that of the thiazole C-5 proton, which is distinctly different in the two isomers and allows the presence of small traces of the other isomer to be detected. In 10% methanol-chloroform, the first and second eluted isomers have $R_f$ of 0.40 and 0.35 respectively on silica gel plates.

Following hydrogenation, the isomeric final penicillins were differentiated by their n.m.r. spectra as follows:

<u>Penicillin from first eluted precursor</u>

δ ($[CD_3]_2SO$), 1.08 (3H, t, J 7Hz, C<u>H</u>$_3$CH$_2$NO, 1.46 and 1.55 (6H, 2s, $(CH_3)_2C$), 3.93 (1H, s, 3-H), 5.2-5.5 (3H, m, 5-H, 6-H and ArC<u>H</u>(NH)CO, 6.50 (1H, s, thiazole 5-H), 7.0 (2H, brs, $D_2O$ exch, thiazole 2-$NH_2$), 8.70 (1H, brd, J 7Hz, $D_2O$ exch, 6-NH), 9.60 (1H, brd, J 7Hz, $D_2O$ exch), ArCH(N<u>H</u>)CO). Other signals are obscured by solvent peaks.

<u>Penicillin from second eluted precursor</u>

δ ($[CD_3]_2SO$ + $D_2O$) 1.08 (3H, t, C<u>H</u>$_3$CH$_2$N), 1.42 and 1.51

(6H, 2s, $(CH_3)_2C$), 3.95 (1H, s, 3-H), 3.2-3.6 (3H, m, 5-H, 6-H and ArC<u>H</u>(NH)CO), 6.52 (1H, s, thiazole 5-H).

BIOLOGICAL DATA

MIC values (μg/ml) for the compound of Example 1.

| ORGANISM | AGAR | BROTH |
| --- | --- | --- |
| E. coli ESS | <0.02 | |
| E. coli JT 4 | >100 | |
| E. coli JT 425 | 25 | |
| E. coli NCTC 10418 | 1.0 | 1.0 |
| Ps. aeruginosa NCTC 10662 | 5.0 | 10 |
| Ps. aeruginosa NCTC 10662 $10^{-2}$ | 2.5 | 25 |
| Ps. aeruginosa Dalgleish $10^{-2}$ | >100 | |
| S. marcescens US 32 | 2.5 | |
| K. aerogenes A | 10 | 25 |
| E. cloacae N1 | 0.5 | |
| P. mirabilis C 977 | 0.2 | |
| P. mirabilis 889 | >100 | |
| P. morganii | 0.5 | |
| P. rettgeri | 2.5 | |
| B. subtilis | 2.5 | |
| S. aureus Oxford | 1.0 | 1.0 |
| S. aureus Russell | 100 | 100 |
| N. catarrhalis 1502 | <0.02 | |
| S. faecalis I | 25 | |
| S. pyogenes CN 10 | 0.1 | |

Claims

1. A compound of formula (I) or a pharmaceutically acceptable salt or in vivo hydrolysable ester thereof:

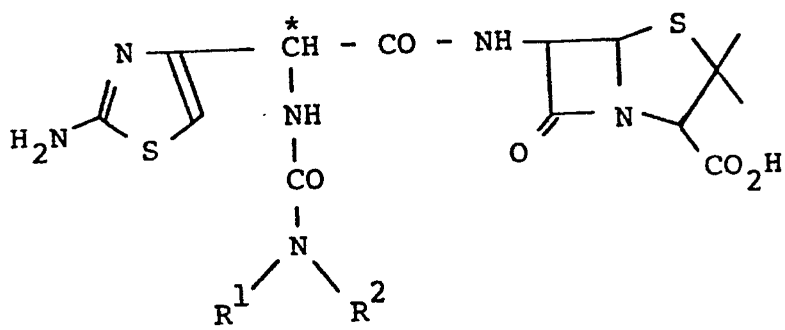

(I)

wherein $R^1$ is hydrogen and $R^2$ is an optionally substituted five- or six-membered heterocyclic group containing one or two nitrogen heteroatoms; or $R^1$ and $R^2$ together with the nitrogen atom to which they are attached form an optionally substituted five- or six-membered heterocyclic group containing one or two nitrogen heteroatoms.

2. A compound as claimed in claim 1 wherein the carbon atom marked * in formula (I) is in the D configuration.

3. A compound as claimed in claim 1 or claim 2 of of formula (II) or a pharmaceutically acceptable salt or in vivo hydrolysable ester thereof:

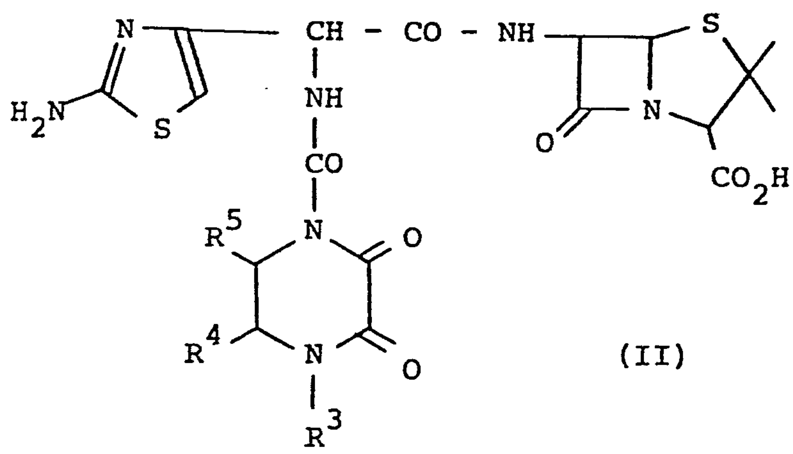

(II)

wherein $R^3$ represents hydrogen or $C_{1-6}$ alkyl; $R^4$ and $R^5$ are the same or different and represent hydrogen, $C_{1-6}$ alkyl, halogen, amino, hydroxy or $C_{1-6}$ alkoxy.

4. A compound as claimed in claim 3 wherein $R^3$, $R^4$ and $R^5$ may be the same or different and each represents methyl, ethyl, n- and iso-propyl, n, sec-, iso- and tert-butyl.

5. A compound as claimed in claim 3 or claim 4 wherein $R^3$ is ethyl.

6. A compound as claimed in any one of claims 3 to 5 wherein $R^4$ and $R^5$ are hydrogen.

7. A compound as claimed in claim 1 selected from the following:

6-[DL-α-(4-ethyl-2,3-dioxopiperazine-1-carbonylamino)2-aminothiazol-4-acetamido]penicillanic acid or a pharmaceutically acceptable salt or in vivo hydrolysable ester thereof; and 6-[D-α-(4-ethyl-2,3-dioxopiperazine-1-carbonylamino)-2-aminothiazol-4-acetamido]penicillanic acid or a pharmaceutically acceptable salt or in vivo hydrolysable ester thereof.

8. A process for the preparation of a compound as claimed in claim 1 which process comprises:

a) reacting a compound of formula (III):

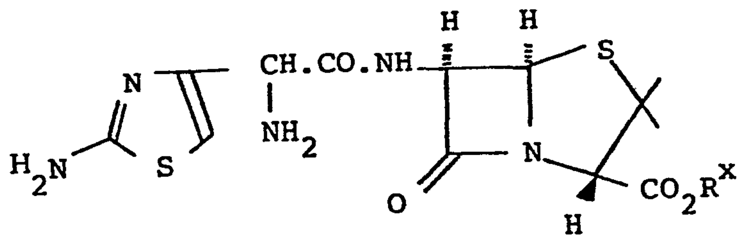

(III)

wherein the α-amino group is optionally substituted with a group which permits acylation to take place, and any reactive groups on the aminothiazole group may be protected, and $R^x$ is hydrogen or a carboxyl-blocking group, with an N-acylating derivative of an acid of formula (IV):

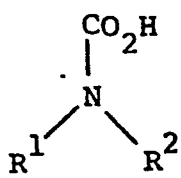

(IV)

wherein $R^1$ and $R^2$ are as defined with respect to formula (I) above and any reactive groups may be protected; or

b) reacting a compound of formula (V):

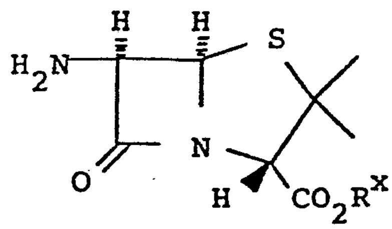

(V)

wherein the amino group is optionally substituted with a group which permits acylation to take place and $R^x$ is as defined with respect to formula (II) above, with an N-acylating derivative of an acid of formula (VI):

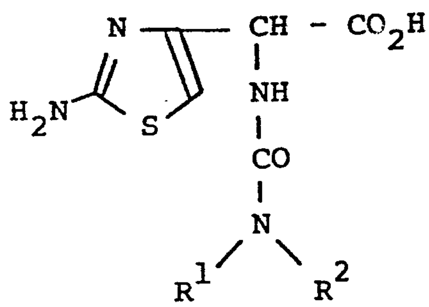

(VI)

wherein $R^1$ and $R^2$ are as defined with respect to formula (I) and any reactive groups therein may be protected; and after processes a) or b), if necessary, carrying out one or more of the following steps:

(i) removing any carboxyl-blocking group $R^x$;
(ii) removing any protecting groups on the side chain group;
(iii) converting the product into a salt or <u>in vivo</u> hydrolysable ester thereof.

9. A pharmaceutical composition comprising a compound as claimed in claim 1 together with a pharmaceutical carrier or excipient.

10. A composition as claimed in claim 9 which further comprises a $\beta$-lactam inhibitor.

# 0066406

European Patent Office

## EUROPEAN SEARCH REPORT

Application number

EP 82 30 2511

**DOCUMENTS CONSIDERED TO BE RELEVANT**

| Category | Citation of document with indication, where appropriate, of relevant passages | Relevant to claim | CLASSIFICATION OF THE APPLICATION (Int. Cl. 3) |
|---|---|---|---|
| Y | EP-A-0 003 992 (BAYER)<br>* Pages 35-37; pages 41-43; example 7c and claims *<br>--- | 1-3,8, 9 | C 07 D 499/70<br>A 61 K 31/43 //<br>C 07 D 277/46 |
| Y | EP-A-0 000 392 (BAYER)<br>* Pages 25,26; claims *<br>--- | 1,8,9 | |
| Y | US-A-4 189 482 (U.D. TRUENER)<br>* Columns 2,3; columns 10,11 (No. 13); claims *<br>--- | 1,8,9 | |
| A | US-A-4 087 424 (I. SAIKAWA)<br>* Columns 2,3, lines 1-37; columns 7,8 *<br>----- | 1,8,9 | |
| | | | TECHNICAL FIELDS SEARCHED (Int. Cl. 3) |
| | | | C 07 D 499/00<br>A 61 K 31/00 |

The present search report has been drawn up for all claims

| Place of search | Date of completion of the search | Examiner |
|---|---|---|
| THE HAGUE | 30-08-1982 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document